# EUROPÄISCHE PATENTSCHRIFT

(11) **EP 3 914 309 B1**
(45) Veröffentlichungstag und Bekanntmachung des Hinweises auf die Patenterteilung: **30.09.2026**
(21) Anmeldenummer: 20702748.3
(22) Anmeldetag: 23.01.2020
(51) Int. Cl.: A61M 60/187, A61M 60/191, A61M 60/289, A61M 60/468, A61M 60/531, A61M 60/867, A61M 60/869

(54) **MEDIZINISCHES KAMMERSYSTEM, EINFÜHRSYSTEM UND KIT**
MEDICAL CHAMBER SYSTEM, INTRODUCTION SYSTEM AND KIT
SYSTÈME DE VENTRICULES MÉDICAL, SYSTÈME D'INSERTION ET KIT

(30) Priorität: 24.01.2019 DE 102019101771
(43) Veröffentlichungstag der Anmeldung: 01.12.2021
(73) Patentinhaber: Artract Medical Inc., New York NY 10175 (US)
(72) Erfinder: GOETZ, Wolfgang, 93051 Regensburg (DE)
(74) Vertreter: Bobbert & Partner Patentanwälte PartmbB
(86) Internationale Anmeldenummer: PCT/EP2020/051633
(87) Internationale Veröffentlichungsnummer: WO 2020/152273

(56) Entgegenhaltungen:
- US-A- 4 957 477
- US-A- 5 385 528
- US-A1- 2002 065 449
- US-A1- 2008 183 286
- US-A1- 2014 194 669
- US-A1- 2015 148 590

## Beschreibung

Die Erfindung bezieht sich auf ein medizinisches Kammersystem und ein Kit. Für die Versorgung von Patienten mit einer Herzinsuffizienz sind Systeme zur Herzunterstützung bzw. zur Unterstützung der Herzaktion, wie z. B. aus der US 2017/0368246 A1, EP 2 482 865 B1 oder US 2018/0193147 A1, bekannt. Beispielsweise gibt es linksventrikuläre und/oder rechtsventrikuläre Herzunterstützungssysteme, die aktiv angetrieben werden können. Die Systeme können vollständig implantiert werden.

Weiterer relevanter Stand der Technik ist beispielsweise in den Dokumenten US4957477 A, US2002/065449 A1 und US2008/183286 A1 offenbart.

Es ist die Aufgabe der vorliegenden Erfindung, ein weiteres System sowie ein Kit umfassend das System und ein Einführsystem für das System bereitzustellen.

Die Aufgabe kann durch das medizinische Kammersystem des Anspruchs 1 gelöst werden und durch das Kit des Anspruchs 6.

Die Aufgabe kann durch das Bereitstellen eines medizinischen Kammersystems (im Folgenden auch kurz: Kammersystem) zur Implantation in den Brustkorb eines Patienten zur Unterstützung der Herzaktion gelöst werden, mit welchem vorzugsweise die Herzspitze des Patienten verlagert bzw. verschoben werden kann.

Das medizinische Kammersystem umfasst wenigstens eine erste Kammer, vorgesehen zu ihrem Anordnen innerhalb eines Herzbeutels des Patienten, und eine zweite Kammer, vorgesehen zu ihrem Anordnen außerhalb des Herzbeutels. Diese beiden Kammern weisen wenigstens einen Verbindungsabschnitt oder einen Verbindungskanal auf, der die Lumen oder jeweils das Innere der beiden Kammern miteinander verbindet. Die Kammern sind ausgestaltet, um mit einem Fluid befüllt zu werden.

Die beiden Kammern sind vorzugsweise vorgesehen und ausgestaltet, um im implantierten Zustand des Kammersystems so angeordnet zu sein, dass die Herzaktion Fluid durch Aufbringung von Druck aus der ersten Kammer verdrängt und dabei in die zweite Kammer überführt. Die zweite Kammer wirkt dabei vorzugsweise als Volumenspeicher und/oder Energiespeicher für das Fluid oder Teile hiervon innerhalb des Kammersystems.

Die Kammern können vorgesehen sein, um bei Einwirken auf sie mittels Drucks auf ihre Kammerwände eine nicht durch ein Bauteil aktiv unterstützte oder bewirkte Volumenverschiebung zwischen einer der beiden Kammern und der anderen dieser Kammern und/oder, nach Implantation des Kammersystems, zwischen einem Raum im Herzbeutel und einem Raum außerhalb des Herzbeutels zu ermöglichen. Hierdurch kann vorzugsweise die Verlagerung der Herzspitze und damit eine axiale Verkürzung der Herzkammern erreicht oder unterstützt werden.

Das erfindungsgemäße Kammersystem ist vorzugsweise dazu ausgestaltet, eine Volumenverschiebung zwischen einem Raum innerhalb des Herzbeutels und einem Raum außerhalb des Herzbeutels zu ermöglichen, die durch die Volumenänderung der Herzkammern betrieben wird.

Die Offenbarung betrifft weiter ein Einführsystem für das Kammersystem. Das Einführsystem dient der Implantation des erfindungsgemäßen Kammersystems in den Patienten. Das Einführsystem kann einen oder mehrere der folgenden Bestandteile umfassen: Einführungskatheter, Führungskatheter, Führungsdraht, Dilatator und Abgabekatheter.

Der Querschnitt des Einführungskatheters und/oder des Lumens kann rund, oval oder polygonal sein. Dabei kann das Einführsystem in einigen Ausführungsformen auch mehrere Exemplare der jeweiligen Katheter und/oder des genannten Führungsdrahts umfassen.

Die Erfindung betrifft zudem ein Kit, das ein erfindungsgemäßes Kammersystem sowie ein Einführsystem umfasst.

Im Sinne der vorliegenden Erfindung kann ein Patient ein Mensch oder ein Tier sein.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlworte.

Soweit im Folgenden nicht anders ausgeführt, bedeutet proximal zur Körpermitte hin und distal von der Körpermitte weg.

Vorteilhafte Weiterentwicklungen der vorliegenden Erfindung sind jeweils Gegenstand von Unteransprüchen und Ausführungsformen.

Wenn hierin von einer Ausführungsform die Rede ist, so ist damit eine exemplarische, erfindungsgemäße Ausführungsform zu verstehen, die nicht als beschränkend zu verstehen ist.

Zuvor beschriebene Systeme zur aktiven Unterstützung der Herzfunktion, siehe z. B. o. g.

US 2017/0368246 A1, EP 2 482 865 B1 oder
US 2018/0193147 A1, unterstützen die Herztätigkeit und komprimieren die Herzkammern durch ein aktives Bauteil, etwa ein mechanisches Bauteil. Dieses Bauteil kann ein Motor sein oder umfassen, der hydraulisch, elektrisch, magnetisch, elektromagnetisch, mechanisch oder dergleichen direkt oder indirekt auf den Herzmuskel wirkt und/oder diesen berührt.

Im Gegensatz hierzu betreibt das erfindungsgemäße Kammersystem vorzugsweise keine aktive mechanische Unterstützung der Herztätigkeit mit einem aktiven mechanischen Bauteil und/oder komprimiert nicht die Herzkammern und/oder weist, insbesondere hierzu, keine Vorrichtung auf, insbesondere keinen Motor, der hydraulisch, elektrisch, magnetisch, elektromagnetisch, mechanisch oder dergleichen direkt oder indirekt auf den Herzmuskel wirkt und/oder eine Pumpwirkung hat, und/oder keine Steuerung oder Steuervorrichtung eingerichtet zum Einwirken auf einen Motor, und/oder ist nicht mit einem solchen Motor oder einer solchen Steuerung oder Steuervorrichtung in Signalübertragung verbunden.

Das erfindungsgemäße Kammersystem kann vorzugsweise die physiologische Verkürzung der langen Achse der Herzkammern im Herzzyklus wieder herstellen oder hierzu beitragen, indem die natürliche Herztätigkeit das im Kammersystem enthaltene Fluid von der ersten Kammer in die zweite Kammer und wieder zurückbewegt und so die Verlagerung der Herzspitze hin zur Herzbasis und wieder zurück ermöglicht oder unterstützt wird.

Die erste Kammer und die zweite Kammer (hierin zusammen auch kurz: Kammern) können optional Druckkammern, Druckausgleichskammern oder dergleichen sein und/oder als solche bezeichnet werden.

Die Kammern können jeweils ein Volumen, einen Diameter und/oder eine lange und eine kurze Achse aufweisen. Die Kammern können gleich oder verschieden voneinander ausgestaltet sein.

Die Kammern können im Gebrauch unterschiedliche Formen annehmen und sich optional den umgebenden anatomischen Strukturen und/oder den wechselnden Druckverhältnissen anpassen. Dabei können die Kammern jeweils, insbesondere hinsichtlich ihrer Größe, Form und/oder Volumen bestimmungsgemäß von einem größeren oder mehr gefüllten Zustand in einen kleineren oder weniger gefüllten Zustand und wieder zurück in einen größeren Zustand überführt werden. Dies geschieht vorzugsweise periodisch, besonders vorzugsweise synchron zur Herzaktion und/oder von dieser bewirkt.

Das Kammersystem weist wenigstens eine Kammerwand auf, optional für jede der Kammern eine oder genau eine eigene Kammerwand. So können eine Kammerwand der ersten Kammer mit einer Kammerwand der zweiten Kammer gemeinsam einstückig vorliegen oder gefertigt sein, oder die Kammerwand der ersten Kammer kann mit der Kammerwand der zweiten Kammer verbunden sein, z. B. mittels oder unter Einbezug einer nachstehend weiter erläuterten Stützvorrichtung.

In einigen erfindungsgemäßen Ausführungsformen ist wenigstens eine Kammerwand vollständig oder zumindest abschnittsweise flexibel verformbar.

Die Kammern sind derart ausgestaltet, dass sich im implantierten Zustand des Kammersystems die erste Kammer im Herzbeutel befindet und die zweite Kammer außerhalb des Herzbeutels befinden kann. Die erste Kammer innerhalb des Herzbeutels ist über Kanäle, vorzugsweise einen Verbindungskanal, mit der zweiten Kammer außerhalb des Herzbeutels vorzugsweise so verbunden, dass Fluid aus der ersten Kammer im Herzbeutel in die zweite Kammer außerhalb des Herzbeutels und wieder zurückfließen kann. Ventile oder andere Mittel zum Regeln oder Steuern des Stroms zwischen den Kammern sind nicht erforderlich und in einigen Ausführungsformen auch nicht vorgesehen. In anderen Ausführungsformen sind Ventile oder andere einstellbare Mittel zum Beeinflussen des Stroms zwischen den Kammern hingegen vorgesehen.

Die Verschiebung des Volumens von einer Kammer in die andere wird durch die Herzaktion im Herzbeutel betrieben. Bei Füllung des Herzens wird durch die Vergrößerung des Herzvolumens im Herzbeutel das Fluid vorzugsweise von der ersten Kammer im Herzbeutel in die zweite Kammer außerhalb des Herzbeutels verschoben. Bei Kontraktion des Herzens wird durch die Verkleinerung des Herzvolumens im Herzbeutel das Fluid vorzugsweise von der zweiten Kammer außerhalb des Herzbeutels in die erste Kammer innerhalb des Herzbeutels verschoben. Durch die Volumenänderung der ersten Kammer innerhalb des Herzbeutels kann sich die Herzspitze synchron zur Herzaktion bei Vergrößerung des Volumens der ersten Kammer im Herzbeutel hin zur Herzbasis bewegen und bei oder unter Verkleinerung der ersten Kammer im Herzbeutel weg von der Herzbasis bewegen. Durch die Verlagerung der Herzspitze wird die lange Achse der Herzkammern während der Systole verkürzt und während der Diastole verlängert.

In einigen Ausführungsformen weist das Kammersystem mehr als zwei Kammern auf. Dabei kann sich beispielsweise die erste Kammer im Herzbeutel befinden, einer oder mehrere weitere Kammern können sich in der rechten Brusthöhle und/oder in der linken Brusthöhle und/oder im Bauchraum befinden. Eine weitere Kammer kann sich in anderen Körperstrukturen, so zum Beispiel unter der Haut befinden. Eine weitere Kammer kann sich außerhalb des Körpers befinden. Eine weitere Kammer kann die Atmosphäre sein. Dabei können unterschiedlich lange Verbindungskanäle zwischen den Kammern, die im Herzbeutel angeordnet sind, und jenen Kammern, die außerhalb des Herzbeutels angeordnet sind, existieren.

Vorzugsweise sind die Kammern im Gebrauch derart implantiert, dass bei der Systole des Herzens Fluid von der zweiten Kammer außerhalb des Herzbeutels in die erste Kammer im Herzbeutel fließt und bei der Diastole Fluid von der ersten Kammer im Herzbeutel in die zweite oder weitere(n) Kammer(n) außerhalb des Herzbeutels fließt. Die Veränderung des Volumens bzw. der Größe der ersten Kammer im Herzbeutel ermöglicht eine Verlagerung der Herzspitze vorzugsweise in axialer Richtung und damit eine Verkürzung der langen Achse der Herzkammern.

Das erfindungsgemäße Kammersystem kann in einigen Ausführungsformen vollständig chirurgisch über eine Eröffnung des Brustkorbes und/oder Bauchraums und/oder eine offene Herzoperation implantiert werden und hierzu ausgestaltet sein. In einigen Fällen kann das Kammersystem über einen Katheter eingesetzt werden und entsprechend ausgestaltet sein. Alternativ ist auch eine Kombination von offener Operation und kathetergestütztem Verfahren möglich.

Das im Gebrauch des Kammersystems in den Kammern vorgesehene Fluid kann jedes Fluid sein, dass über die notwendigen Flusseigenschaften verfügt.

Vorzugsweise hat der Verbindungskanal, der die erste Kammer mit der zweiten Kammer verbindet, einen Durchmesser von 0,5 cm - 5 cm Durchmesser, besonders vorzugsweise einen Durchmesser von 15 mm - 20 mm. Der Verbindungskanal, der diese Kammern miteinander verbindet, hat vorzugsweise eine Länge von 1 mm - 500 mm, besonders vorzugsweise eine Länge von 1 mm - 100 mm, ganz besonders vorzugsweise eine Länge von 2 mm - 10 mm.

Die Kammern können bereits vor Implantation des Kammersystems mit Fluid befüllt sein. Das Fluid besteht vorzugsweise aus einem Fluid von geringer Viskosität, vorzugsweise aus einem Gemisch aus Flüssigkeiten, besonders vorzugsweise Wasser (H₂O) aufweisend und/oder mit isotoner Eigenschaft. Weiter kann das Fluid besonders vorzugsweise Luft sein oder umfassen, besonders vorzugsweise aus einem definierten Gasgemisch bestehen. Das Gas oder Gasgemisch kann besonders vorzugsweise Helium sein oder aufweisen.

Die Kammern können in einigen erfindungsgemäßen Ausführungsformen nach erfolgter Implantation, also wenn sie sich bereits im Körper befinden und dort angeordnet sind, mit Fluid befüllt werden. Sie können entsprechend vorbereitet oder ausgestaltet sein, **z. B.** eine - vorzugweise mittels Verschlussvorrichtung verschließbare - Öffnung aufweisen. Diese Anordnung im Körper kann eine vorläufige oder eine endgültige Position der Kammern im Körper sein. Ein nach Anordnung oder Implantation erfolgendes Befüllen oder Korrigieren des Grades an Befüllung oder des in den Kammern enthaltenen Fluidvolumens kann vorteilhaft eine Anpassung des Kammersystems an die Bedürfnisse des konkreten Patienten ermöglichen, die in einigen Ausführungsformen auch zu einem deutlich nach der Implantation liegenden Zeitpunkt erfolgen kann.

Die Verlagerung des Fluids von einer Kammer in die verbundene andere Kammer wird in manchen offenbarten Ausführungsformen allein durch die Veränderung des Herzvolumens innerhalb des Herzbeutels betrieben oder bewirkt.

Erfindungsgemäß umfasst das Kammersystem eine Stützvorrichtung. Die wenigstens zwei Kammern, die erste und zweite Kammer, bilden gemeinsam mit dem Verbindungskanal genau einen geschlossenen Beutel aus, wobei die Stützvorrichtung den Verbindungskanal zumindest abschnittsweise ausformt oder ihm Struktur gibt. Die Stützvorrichtung kann ein Hohlkörper, z. B. eine, z. B. flexibel, verformbare, Stützvorrichtung, insbesondere ein Stent, ein Ring oder ein Zylinder sein.

In einigen Ausführungsformen wird der Durchmesser des Verbindungskanals zwischen den Kammern durch eine - sich nach ihrer Implantation vorzugsweise ausdehnende - Vorrichtung, die mit der Wand des Verbindungskanals verbunden sein kann, konstant gehalten. Vorzugsweise ist diese Vorrichtung ein Stent. Besonders vorzugsweise hält der Stent die Öffnung im Herzbeutel in einer optional vorgegebenen Größe offen. Besonders vorzugsweise besteht der Stent aus Memory Material (Nitinol oder Polymer) oder weist ein solches Material auf.

In einigen Ausführungsformen besteht der Verbindungskanal, der die Kammern verbindet, aus einem elastischen Polymer oder Polymerfilm, der sich vorzugsweise dem Durchmesser des Kanals anpasst.

In einigen Ausführungsformen bestehen die Kammerwände (auch: die Kammerwand) aus einem elastischen Polymerfilm, der sich dem veränderlichen Volumen des in der Kammer vorliegenden Fluids anpasst, oder weisen einen solchen auf.

Vorzugsweise ist das Kammersystem zur zumindest abschnittsweisen Implantation in den Herzbeutel und den Brustkorb und/oder den Bauchraum ausgestaltet. Die Vorrichtung ist vorzugsweise derart ausgestaltet, dass sie zumindest teilweise in einen Herzbeutel implantiert werden kann.

In einigen Ausführungsformen ist die Vorrichtung derart ausgestaltet, dass ein Abschnitt im Herzbeutel und/oder der Brusthöhle und/oder in einem weiteren Abschnitt im Bauchraum implantiert werden kann.

In manchen Ausführungsformen des erfindungsgemäßen Kammersystems ist diese derart gestaltet, dass sich mehrere Kammern zur gleichen Zeit im Herzbeutel, im Brustkorb und/oder im Bauchraum und/oder außerhalb des Körpers befinden können.

Vorzugsweise befindet sich die erste Kammer im Herzbeutel und ist mittels des Verbindungskanals mit der zweiten Kammer in Fluidverbindung verbunden.

In einigen Ausführungsformen ist die zweite Kammer die Atmosphäre oder hat einen Fluidverbindung hiermit. In einigen Ausführungsformen befindet sich die zweite Kammer nach Implantation bestimmungsgemäß außerhalb des Körpers des Patienten.

In manchen Ausführungsformen befindet sich die zweite Kammer nach Implantation bestimmungsgemäß innerhalb des Körpers.

In einigen Ausführungen befindet sich die zweite Kammer nach Implantation bestimmungsgemäß im Bauchraum. In einigen Ausführungsformen befindet sich die zweite Kammer nach Implantation bestimmungsgemäß in der rechten Brusthöhle.

In manchen Ausführungen befindet sich die zweite Kammer nach Implantation bestimmungsgemäß in der linken Brusthöhle.

In einigen Ausführungen befinden sich mehrere zweite Kammern nach Implantation bestimmungsgemäß innerhalb und/oder außerhalb des Körpers.

In manchen Ausführungsformen weist das erfindungsgemäße Kammersystem einen Stent im oder am Abschnitt des Kanals, der die Kammern verbindet, auf. Ein solcher Stent kann in einigen Ausführungsformen der einzige Abschnitt des Kammersystems sein, welches ein Metall oder eine Legierung aufweist.

In manchen Ausführungsformen besteht das Kammersystem aus den Kammern und ihren Verbindungskanälen, erfindungsgemäß ferner aus der Stützvorrichtung und optional ferner aus einem Stützgerüst zum Unterstützen der Form der Kammern.

In einigen Ausführungsformen bilden die Kammern gemeinsam ein abgeschlossenes Fluidsystem. Dieses weist, zumindest im Gebrauch, vorzugsweise keine Zu- oder Ableitung auf und/oder ist vorzugsweise nicht mit einem Äußeren der Kammer und/oder des Patienten fluidisch verbunden.

In manchen Ausführungsformen weisen die Kammern eine vorgegebene Form auf, in anderen nicht.

In einigen Ausführungsformen wird die Form wenigstens einer der Kammern durch ein Gerüst unterstützt.

In manchen Ausführungsformen wird die Form wenigstens einer der Kammern durch ein Gerüst mit

Memoryeigenschaften oder Formgedächtniseigenschaften unterstützt.

In einigen Ausführungsformen wird die Form wenigstens einer der Kammern durch einen Stent unterstützt.

In manchen Ausführungsformen können die Kammern durch eine eigene elastische Kraft eine Flussrichtung des Fluids in oder zwischen den Kammern unterstützen.

In einigen Ausführungsformen weisen wenigstens zwei Kammern, z. B. die erste Kammer und die zweite Kammer oder weitere Kammern, des erfindungsgemäßen Kammersystems, oder ihre Kammerwände, voneinander verschiedene Elastizitäten, Elastizität-Module (E-Module, Dehnungsmodule, oder Youngsche Module) und/oder Innenvolumen (z. B. in einem Zustand ohne Flüssigkeit und/oder ohne äußere Einwirkung, oder in einem entspannten Zustand) auf.

Das erfindungsgemäße Kammersystem unterstützt vorzugsweise die Verlagerung der Herzspitze in Richtung zur Herzbasis. Die Vorrichtung unterstützt dadurch die Verkürzung der langen Achse des Herzens. Die Vorrichtung vergrößert dadurch das Blutvolumen, das durch die Verkürzung der langen Achse vom Herzen ausgeworfen werden kann.

In einigen Ausführungsformen umfasst das Kammersystem Sensoren oder ist mit Sensoren verbunden. Die Sensoren können Drucksensoren und/oder Lagesensoren sein. Die Sensoren sind dabei vorzugsweise dazu vorgesehen und geeignet, physiologische Parameter zu messen, die mit der Füllung der Kammern, dem Druck in den Kammern, dem Fluss des Fluids und/oder der Herzaktivität in Bezug oder Verbindung stehen. Die Sensoren sind dabei vorzugsweise derart ausgestaltet, dass sie ein Sensorsignal bereitstellen, das einer Vorrichtungen im Körper des Patienten und/oder außerhalb dessen Körpers übermittelt wird, z. B. drahtlos oder drahtgebunden. Entsprechende Sender und/oder Empfänger für das Signal können vorgesehen sein.

Die solche Sensorsignale erhaltende Vorrichtung kann eine Steuereinheit des Kammersystems sein, die mit wenigstens einer Einrichtung zum Steuern oder Regeln des Flusses oder Durchflusses des Fluids zwischen den Kammern verbunden ist. Zu solchen Einrichtungen zum Steuern oder Regeln des Durchflusses des Fluids zwischen den Kammern, wobei diese Einrichtungen Teil des Kammersystems sein können, können Ventile, Drosseln und andere, einstellbare Fluss- oder Strömungsbegrenzer wie Blenden usw. zählen.

Das erfindungsgemäße Kammersystem kann somit Aktoren aufweisen, die z. B. auf Einrichtungen zum Steuern oder Regeln des Durchflusses des Fluids zwischen den Kammern, welche z. B. im Verbindungskanal oder in der Stützvorrichtung vorgesehen sein können, einzuwirken. Diese Einrichtungen, zu deren Betätigung eine Antriebseinheit im Kammersystem vorgesehen sein kann, das z. B. aus einer Batterie oder auf andere Weise mit Energie gespeist wird, wirken vorzugsweise jedoch nicht auf den Herzmuskel ein, weshalb sie im Sinne der vorliegenden Erfindung kein Motor zum Einwirken auf die Herzfunktion sind, die Herztätigkeit nicht unterstützen und/oder die Herzkammern nicht oder nicht aktiv unterstützen.

In einigen Ausführungsformen umfasst das erfindungsgemäße Kammersystem Sensoren, welche die elektrische Herzaktivität messen, Drucksensoren (die beispielsweise den Blutdruck in Gefäßen und/oder in einer oder mehreren Herzkammern messen), Sensoren für Kraft, elektrische Spannung und/oder elektrischen Strom, Druck und/oder für das Ausmaß der Formveränderung wenigstens einer der Kammern, oder ist mit solchen Sensoren verbunden.

In einigen Ausführungsformen umfasst das erfindungsgemäße Kammersystem eine Vorrichtung und/oder Sensoren zum Erhalten eines EKG-Signals (Elektrokardiogramm).

In manchen Ausführungsformen ist vorgesehen, die Einrichtungen zum Steuern oder Regeln des Durchflusses des Fluids zwischen den Kammern, synchron zur Herzaktion zu steuern. Das Kammersystem kann entsprechend programmiert sein.

Während des gesamten Herzzyklus bleibt normalerweise die Herzspitze annähernd stationär, was unter anderem durch den Herzbeutel verursacht wird. Der Herzbeutel ist ein geschlossener Sack, der circa 10 ml Flüssigkeit enthält und das gesamte Herz umfasst. Der geschlossene Herzbeutel ist flüssigkeitsdicht und erlaubt somit keinen Flüssigkeitsaustausch mit den umgebenden Körperhöhlen. Bei der Kontraktion des Herzens kann der zum Teil frei im Brustkorb liegende Herzbeutel der Verringerung des Herzumfangs folgen. Der Herzbeutel ist jedoch im Bereich der Herzspitze über das sterno-perikardiale Ligament mit dem Sternum und dem Processus xiphoideus verbunden und liegt fest verbunden dem Zwerchfell auf. Bei der Kontraktion des Herzens können sich der Herzbeutel und die in dem geschlossenen Herzbeutel liegende Herzspitze nicht von der Brustwand hin zur Herzbasis bewegen. Aus diesem Grund führen die Kontraktion des Herzmuskels und die Verkürzung des Herzens zu einem Zug an der Herzbasis und der elastischen Aortenwurzel, die auf diese Weise in der Systole gestreckt wird. Sobald sich der Herzmuskel in der Diastole entspannt, zieht die gestreckte Aortenwurzel durch die in ihr gespeicherte Spannenergie die Herzbasis weg von der stationären Herzspitze. Bei Untersuchungen mit Schafen, die in Bezug auf das Herz eine mit dem Menschen vergleichbare Anatomie aufweisen, zeigt sich, dass in Ruhe durch die Spannung eine Kraft zwischen Aortenwurzel und Herzbasis von 1,8 ± 0,2 N besteht. Um die Aortenwurzel 10 mm in Richtung der Herzspitze zu bewegen, muss eine Kraft von 1,8 ± 0,1 N aufgewendet werden. Der normale Ausschlag des Hubes der Aortenwurzel beträgt beim gesunden Menschen 12 ± 2 mm. Pro Herzschlag wird damit zur Auslenkung der Aortenwurzel eine Arbeit von etwa W = F * s = 1,8 N * 0,012 m=0,0216 J verrichtet, wobei die entsprechende Energie zum Teil in der elastischen Dehnung der Aortenwurzel gespeichert wird.

Herzschwäche, insbesondere Herzinsuffizienz, besteht, wenn das Herz nur noch weniger als 45% des Herzkammervolumens auswerfen kann. Von einer hochgradigen Einschränkung spricht man bei einer Auswurfleistung von weniger als 35%.

Zu der Symptomatik der Herzinsuffizienz zählt Atemnot (Belastungs-, Ruhedyspnoe, Orthopnoe, paroxysmale nächtliche Dyspnoe), Müdigkeit, inadäquate Erschöpfung nach Belastungen, Schwäche, Lethargie, Flüssigkeitsretention (Bein- oder Bauchschwellung, Gewichtszunahme), nächtliches Wasserlassen (Nykturie), trockener Husten (besonders nachts), Schwindel, Synkopen, Inappetenz, Übelkeit, Völlegefühl, Meteorismus, Obstipation, abdominelle Schmerzen, unter Umständen Gewichtsabnahme, Gedächtnisstörungen, Verwirrtheitszustände und kognitive Beeinträchtigungen.

Die Herzinsuffizienz ist nach Schweregrad eingeteilt in NYHA I (diagnostizierte Herzkrankheit ohne Symptome und ohne Einschränkung der Belastbarkeit), NYHA II (leichte Einschränkung der Belastbarkeit, keine Symptome in Ruhe, sondern erst bei stärkerer Belastung), NYHA III (starke Einschränkung der Belastbarkeit, keine Symptome in Ruhe, jedoch bereits bei leichter Belastung) und NYHA IV (persistierende Symptomatik auch in Ruhe). Bei der Diagnostik der Herzinsuffizienz ist vor allem die Echokardiographie von Bedeutung, mit der regionale und globale Einschränkungen der Herzfunktion beurteilt werden können.

Patienten mit einer Herzschwäche mit einer linksventrikulären Ejektionsfraktion (EF) von weniger als 45 % zeigen einen verringerten Hub der Aortenwurzel auf weniger als 8 mm während der Systole. Die Verkürzung der langen Herzachse ist ein besonders sensibler Parameter für die Messung der Herzfunktion.

Bei einer Herzinsuffizienz mit reduzierter Ejektionsfraktion (HFrEF) wird in der Echokardiographie eine verminderte Wandbewegung des Herzens mit verringerter Auswurffraktion von weniger als 45 % gemessen. Dabei zeigt sich, dass die Verkürzung sowohl der langen Achse von Herzspitze zu Herzbasis als auch der dazu orthogonal gelegenen kurzen Achse verringert ist. Mit zweidimensionaler Echokardiographie (Simpson-Methode) kann die Verringerung des Auswurfvolumens bestimmt werden.

Mindestens die Hälfte der Patienten mit Symptomen der Herzinsuffizienz hat eine in der üblichen Echokardiographie gemessene normale Herzfunktion mit einer normalen Verkürzung des orthogonalen Durchmessers der Herzkammern. Die daraus errechnete Ejektionsfraktion erscheint als normal. Diese Form der Herzinsuffizienz wird als Herzinsuffizienz mit erhaltener Ejektionsfraktion (HFpEF) bezeichnet. Als Ursache der Herzinsuffizienz werden eine Versteifung der Herzmuskulatur und eine beeinträchtigte diastolische Funktion des Herzens diskutiert. Bei genauerer echokardiographischer Betrachtung der Herzfunktion dieser Patienten zeigt sich häufig jedoch eine Veränderung der Ver- und Entwindung der linken Herzkammer sowie eine Verminderung des diastolischen Sogs und der frühen diastolischen Füllung der linken Herzkammer.

Die normale Bewegung der Aortenwurzel mit der Herzbasis hin zur Herzspitze von 12 ± 2 mm ist auf deutlich weniger als 8 mm verringert. Die Geschwindigkeit des Hubes der Aortenwurzel in der Systole und frühen Diastole ist hochsignifikant vermindert mit 0,64 ± 0,51 cm/s im Vergleich zu 1,54 ± 0,51 cm/s in der Systole und 1,49 ± 0,77 cm/s im Vergleich zu 2,32 ± 1,24 cm/s zu Beginn der Diastole. Die mangelnde Leistungsfähigkeit und die Herzinsuffizienzsymptome dieser Patienten korrelieren mit einer geringeren Elastizität, insbesondere einer Versteifung der Aortenwurzel. Gleichzeitig findet sich bei diesen Patienten eine Verdickung des Herzmuskels, was auf eine Kompensation der geringeren Windkesselfunktion und einen geringeren Hub der Aortenwurzel hindeutet. Die Herzkraft erscheint in der Echokardiographie noch normal oder sogar erhöht, reicht jedoch nicht mehr aus, um die Aortenwurzel zur Herzspitze zu ziehen, da diese weniger elastisch ist und eine erhöhte Kraft erforderlich wäre um einen ausreichenden Hub der Aortenwurzel zu bewirken. Die verringerte Verkürzung der langen Achse des Herzens hat eine Verminderung des Schlagvolumens zur Folge.

Das erfindungsgemäße Kammersystem soll bevorzugt verwendet werden bei chronischer Herzinsuffizienz mit erhaltener linksventrikulärer Ejektionsfraktion (HFpEF), bei der die systolische Herzmuskelfunktion weitgehend erhalten ist, doch die Herzkraft nicht mehr ausreicht, eine durch verschiedene Erkrankungen oder durch Alterungsprozesse versteifte Aortenwurzel zu dehnen und damit verbunden einen ausreichenden Hub der Herzbasis bzw. Streckung der Aortenwurzel zu erzeugen.

In einigen erfindungsgemäßen Ausführungsformen umfassen die Kammern des medizinischen Kammersystems wenigstens zwei separate Beutel, die zu einem gemeinsamen Fluidraum direkt oder indirekt verbunden sind. Die Verbindung der beiden separaten Beutel kann eine Hülse sein, die als Verbindungskanal zwischen den beiden Beuteln bezeichnet werden kann. Die beiden Beutel können mittels adapterförmigen Anschlussöffnungen auf die Hülse, insbesondere auf die beiden Hülsenenden, aufgeschoben oder aufgesteckt werden. Zusätzlich können die Beutelöffnungen an der Hülse fixiert werden, beispielsweise mittels einer Naht, einer Klebung oder ähnlichem.

In einigen erfindungsgemäßen Ausführungsformen beträgt das Füllvolumen der Kammern zusammen zwischen 0,1 ml und 100 ml, insbesondere zwischen 1 ml und 50 ml.

In einigen erfindungsgemäßen Ausführungsformen ist das medizinische Kammersystem vollständig oder abschnittsweise steril.

Einige der erfindungsgemäßen Ausführungsformen können die folgenden Vorteile aufweisen:
Das erfindungsgemäße Kammersystem ermöglicht vorteilhaft die Verlagerung der Herzspitze hin zur Herzbasis zur Unterstützung der Herzaktion.

Anders als bei der normalen Verkürzung der langen Herzachse während der Systole durch Verlagerung der Herzbasis hin zur Herzspitze, ermöglicht das erfindungsgemäße Kammersystem vorteilhaft die Verlagerung der Herzspitze hin zur Herzbasis.

Durch die Wiederherstellung der Verkürzung der langen Herzachse während der Systole kann der Auswurf der Blutmenge aus dem Herzen vorteilhaft mittels des erfindungsgemäßen Kammersystems erhöht werden.

Die Wiederherstellung der Verkürzung der langen Herzachse mittels des erfindungsgemäßen Kammersystems führt vorteilhaft zu einer besseren Entleerung der Herzkammern und verringert das endsystolische Volumen im Herzen. Die Herzkammer kann daher in der Diastole mehr Volumen aufnehmen und in der Systole mehr Blut auswerfen, wodurch es zu einer höheren Effektivität der Herzfunktion kommt und der Druck im linken Vorhof gesenkt wird.

Bei Patienten mit HFpEF sind die Dehnung und der Hub der versteiften Aortenwurzel reduziert. Bei diesen Patienten ist die Herzkraft normal oder kompensatorisch vergrößert. Die mangelnde Verkürzung der Herzkammern während der Systole kann nicht durch eine zusätzliche Reduktion des Durchmessers des Ventrikels kompensiert werden und hat eine Verminderung des Auswurfvolumens zur Folge. Das erfindungsgemäße Kammersystem unterstützt vorteilhaft die Verlagerung der Herzspitze, und ersetzt so die nicht mehr mögliche Verlagerung der Herzbasis mit einer Verlagerung der Herzspitze. Dies führt zu einer Verkürzung der Längsachse des Herzens und damit zu einer verbesserten Auswurfleistung des Herzens.

Ein positiver, vorteilhafter Effekt des erfindungsgemäßen Kammersystems auf die Muskelkontraktion der beiden Herzkammern und der beiden Vorhöfe kann ein Nebeneffekt sein.

Im Folgenden wird die erfindungsgemäße Vorrichtung anhand bevorzugter Ausführungsformen derselben unter Bezugnahme auf die angehängten Figuren beschrieben.

Die Erfindung ist jedoch nicht auf diese Ausführungsformen beschränkt, sondern wird durch die beigefügten Ansprüche definiert.

In den Figuren gilt:
- **Fig. 1A und 1B**: zeigen eine Darstellung eines Herzens mit den vier Herzkammern, Herzbasis und Herzskelett;
- **Fig. 2A und 2B**: zeigen eine Darstellung der Pumpfunktion des Herzens;
- **Fig. 3**: zeigt eine Darstellung der Aufhängung des Herzbeutels in Brustkorb;
- **Fig. 4A bis 4C**: zeigen eine Darstellung der Pumpfunktion des Herzens mit stationärer Herzbasis;
- **Fig. 5A und 5B**: zeigen eine schematische Darstellung der normalen Pumpfunktion der linken Herzkammer;
- **Fig. 6A und 6B**: zeigen eine schematische Darstellung der Pumpfunktion der linken Herzkammer mit stationärer Herzbasis;
- **Fig. 7A und 6B**: zeigen eine schematische Ausführungsform des erfindungsgemäßen Kammersystems, die eine Verlagerung der Herzspitze bei stationärer Herzbasis ermöglicht;
- **Fig. 8A bis 8D**: zeigen einen Einführungskatheter zur Platzierung des erfindungsgemäßen Kammersystems;
- **Fig. 9**: zeigt das erfindungsgemäße Kammersystem im Herzbeutel;
- **Fig. 10A und 10B**: zeigen schematisch die Funktion des erfindungsgemäßen Kammersystems; und
- **Fig. 11A und 11B**: zeigen das erfindungsgemäße Kammersystems im Herzbeutel mit Herz.

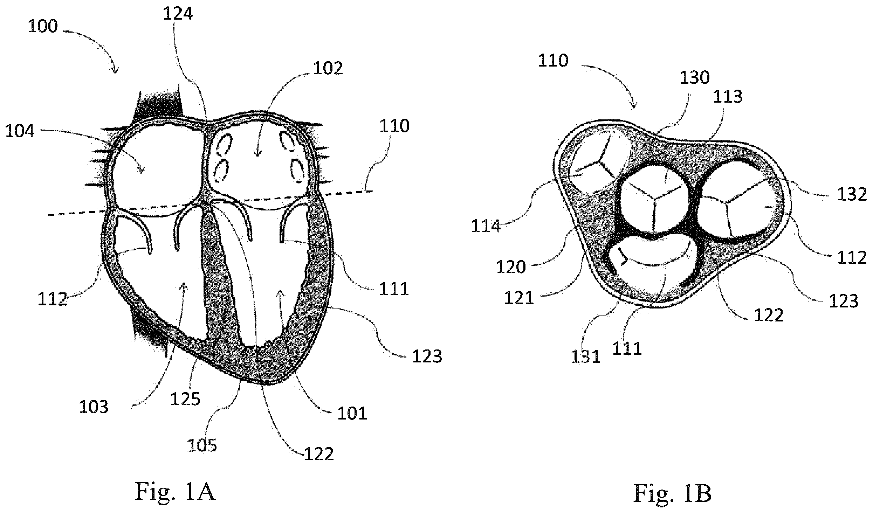

**Fig. 1A und 1B** zeigen ein menschliches Herz 100 mit den vier Herzkammern und der Herzbasis.

In **Fig. 1A** sind der linke Ventrikel 101 (linke Herzkammer) und das linke Atrium 102 (linker Vorhof) sowie die dazwischenliegende Mitralklappe 111, der rechte Ventrikel 103 (rechte Herzkammer) und das rechte Atrium 104 (rechter Vorhof) mit der dazwischenliegenden Trikuspidalklappe 112 dargestellt. Zwischen den beiden Atria befindet sich das Vorhofseptum 124. Zwischen den beiden Ventrikeln 101 und 103 befindet sich das intraventrikuläre Septum 125.

In **Fig. 1B** ist die Herzbasis 110 dargestellt. Die Herzbasis ist eine mehr oder weniger ebene anatomische Struktur des Herzens, in oder an der sich die beiden atrioventrikulären Klappen, nämlich die Mitralklappe 111 und Trikuspidalklappe 112, sowie die beiden Taschenklappen, nämlich die Aortenklappe 113 und die Pulmonalklappe 114, befinden. Das Herzskelett 120 besteht aus knorpeligem Gewebe und ist die einzige steife Struktur des Herzens. Das Herzskelett 120 umfasst vollständig die Aortenwurzel 130 sowie die zentralen Anteile des Mitralklappenrings 131 und des Trikuspidalklappenrings 132. Die am kräftigsten ausgebildeten Anteile des Herzskelettes sind das linke fibröse Trigon 121 und das rechte fibröse Trigon 122. Am rechten fibrösen Trigon 122 grenzen die Mitralklappe 111 und die Trikuspidalklappe 112 aneinander. Der Herzmuskel 123 des interventrikulären Septums 125 ist im Bereich des rechten fibrösen Trigons 122 zwischen Mitralklappe 111 und Trikuspidalklappe 112 mit dem Herzskelett 120 verbunden. An dieser Stelle führt die Kontraktion des Herzmuskels 123 und vor allem des interventrikulären Septums 125 zu einem Zug am Herzskelett 120 und der damit verbundenen Aortenklappe 113 in Richtung Herzspitze.

Die Mitralklappe 111 und Trikuspidalklappe 112 schließen sich am Ende der Diastole und stellen sicher, dass das Blut bei Kontraktion der Ventrikel 101 und 103 nicht zurück in die Atria 102 und 104 fließt, sondern vorwärts gepumpt wird, auf der rechten Seite in die Lungen und auf der linken Seite in den Körperkreislauf. Die Aortenklappe 113 und die Pulmonalklappe 114 schließen sich am Ende der Systole und stellen sicher, dass nach erfolgter Kontraktion der beiden Ventrikel 101 und 103 das Blut nicht zurück in die Ventrikel 101 und 103 fließt, sondern auf der rechten Seite der diastolische Blutdruck in der Pulmonalarterie und der Lunge und auf der linken Seite der diastolische Druck in der Aorta und dem Körperkreislauf aufrechterhalten bleibt.

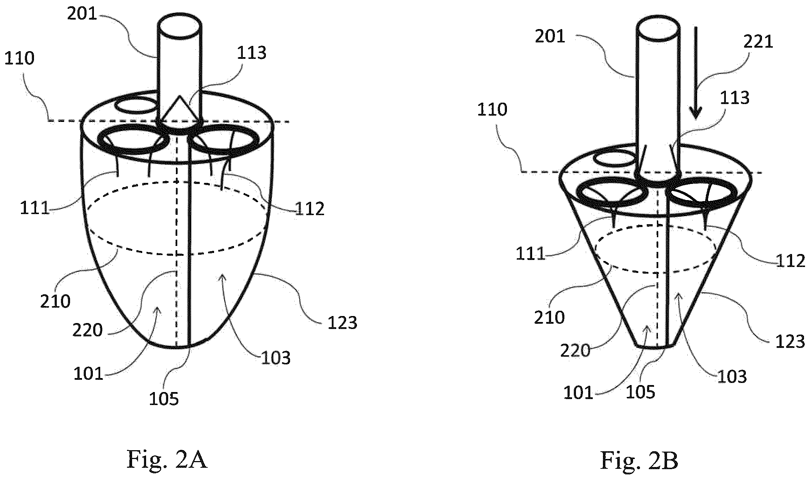

**Fig. 2A und 2B** zeigen eine schematische Darstellung des Herzens.

**Fig. 2A** zeigt das Herz in der Diastole mit entspanntem Herzmuskel 123. Die Längsachse 220 ist hier im Kontraktionszyklus am längsten und der Umfang 210 der Herzkammern 101, 103 und der orthogonale Diameter am größten. Die beiden Herzkammern 101 und 103 sind gefüllt, die Mitralklappe 111 und Trikuspidalklappe 112 sind geöffnet, um den Bluteinstrom in die Herzkammern zu ermöglichen. Die Aortenklappe 113 ist geschlossen und verhindert so den Rückfluss von Blut aus dem Körperkreislauf in die linke Herzkammer und erhält den diastolischen Blutdruck im Körper aufrecht. Die Aortenwurzel 201 ist am Ende der Diastole maximal zusammengezogen.

**Fig. 2B** zeigt schematisch die Kontraktion des Herzens in der Systole mit einer normal elastischen Aortenwurzel 201. Der Herzmuskel 123 ist kontrahiert, das Herz hat den geringsten Umfang 210 und den geringsten orthogonalen Diameter im Herzzyklus. Die Herzspitze 105 verbleibt stationär, die Herzbasis 110 hat sich zur Herzspitze verlagert (Bewegungsrichtung der Herzbasis 221) und die Längsachse 220 ist am kürzesten. Die atrioventrikulären Klappen 111 und 112 sind geschlossen um den Rückfluss von Blut in die beiden Vorhöfe 102 und 104 zu verhindern. Die Aortenklappe 113 ist geöffnet um den Auswurf von Blut in den Körperkreislauf zu ermöglichen. Die Aortenwurzel 201 ist maximal gestreckt.

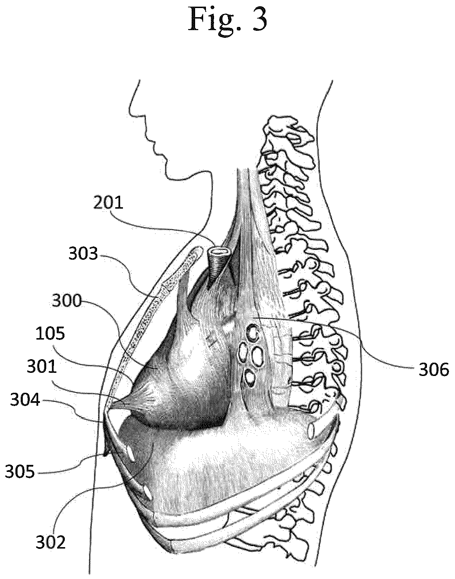

**Fig. 3** zeigt eine schematische Darstellung des Brustkorbes eines Menschen. Der Herzbeutel 300 liegt dem Zwerchfell 302 auf und ist zwischen der Aortenwurzel 201 mit dem Mediastinum 306 und dem sterno-perikardialen Ligament 301 aufgespannt. Das sterno-perikardiale Ligament 301 erstreckt sich vom Herzbeutel 300 im Bereich der Herzspitze 105 zum Ende des Sternums 303 im Bereich des Processus xiphoideus 304 und der Rippe 305.

Bei Kontraktion des Herzens kann der Herzbeutel 300 der Verringerung des Herzumfanges 210 während der Systole folgen. Da der geschlossene Herzbeutel jedoch über das sterno-perikardiale Ligament 301 zwischen dem relativ unbeweglichen Sternum 303 und dem Mediastinum 306 aufgespannt ist, kann sich die Herzspitze 105 nicht vom Sternum 303 weg in Richtung zur Herzbasis 110 verlagern. Aus diesem Grund führt die Kontraktion des Herzens und Verkürzung der Längsachse 220 des Herzens zu einem Zug an der Herzbasis 110 und der elastischen Aortenwurzel 201, die auf diese Weise in der Systole gestreckt und die Herzbasis 101 mit der Aortenwurzel 201 in Richtung Herzspitze gezogen wird.

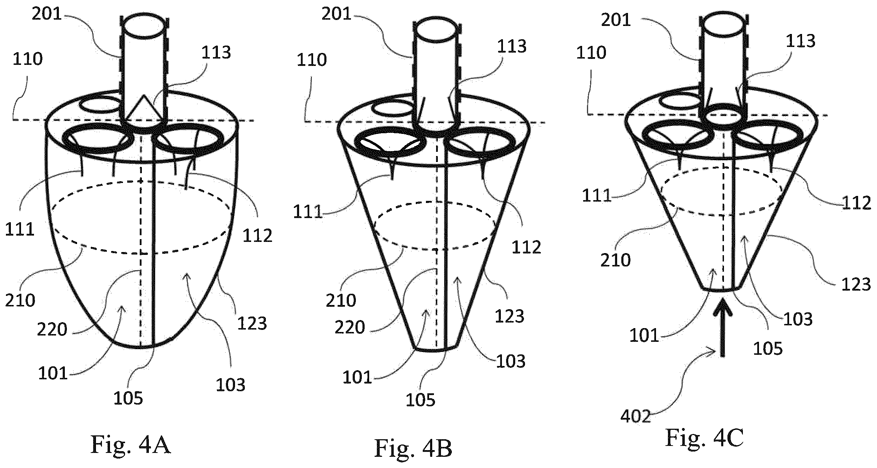

**Fig.4A bis 4C** zeigen schematisch ebenfalls die Kontraktion des Herzens in der Systole, jedoch bei steifer, nicht elastischer Aortenwurzel 201 und stationärer Herzbasis 110.

**Fig. 4A** zeigt das Herz in der Diastole mit entspanntem Herzmuskel 123. Die Längsachse 220 ist hier im Kontraktionszyklus am längsten und der Umfang 210 der Herzkammern 101,103 und der orthogonale Diameter am größten. Die beiden Herzkammern 101 und 103 sind gefüllt, die Mitralklappe 111 und Trikuspidalklappe 112 sind geöffnet, um den Bluteinstrom in die Herzkammern zu ermöglichen. Die Aortenklappe 113 ist geschlossen und verhindert so den Rückfluss von Blut aus dem Körperkreislauf in die linke Herzkammer und erhält den diastolischen Blutdruck im Körper aufrecht.

**Fig. 4B** zeigt das Herz bei kontrahiertem Herzmuskel 123, das Herz hat den geringsten Umfang 210 und geringsten orthogonalen Diameter im Herzzyklus. Die Herzspitze 105 verbleibt stationär, die Lage der Herzbasis 110 hat sich gegenüber der Diastole nicht verändert, da die steife Aortenwurzel 201 nicht gedehnt werden kann und so die Herzbasis nicht zur Herzspitze hingezogen werden kann. Die Längsachse 220 ist ebenso lang wie beim entspannten Herzen in der Diastole in **Fig. 4A****.** Aufgrund der Muskelkontraktion ist jedoch der Umfang 210 und orthogonale Diameter am geringsten im Herzzyklus. Die atrioventrikulären Klappen 111 und 112 sind geschlossen, um den Rückfluss von Blut in die beiden Vorhöfe 102 und 104 zu verhindern. Die Aortenklappe 113 ist geöffnet, um den Auswurf von Blut in den Körperkreislauf zu ermöglichen.

**Fig. 4C** zeigt das Herz bei kontrahiertem Herzmuskel 123 unter Verwendung des erfindungsgemäßen Kammersystems. Die Herzbasis 110 bleibt stationär wie in **Fig. 4B****.** Die Längsachse 220 ist am kürzesten wie in **Fig. 2B****,** da es das erfindungsgemäße Kammersystem ermöglicht, dass sich die Herzspitze 105 in Richtung hin zur Herzbasis 110 (Bewegungsrichtung der Herzspitze 402) bewegen kann. Die atrioventrikulären Klappen 111 und 112 sind geschlossen um den Rückfluss von Blut in die beiden Vorhöfe 102 und 104 zu verhindern. Die Aortenklappe 113 ist geöffnet um den Auswurf von Blut in den Körperkreislauf zu ermöglichen.

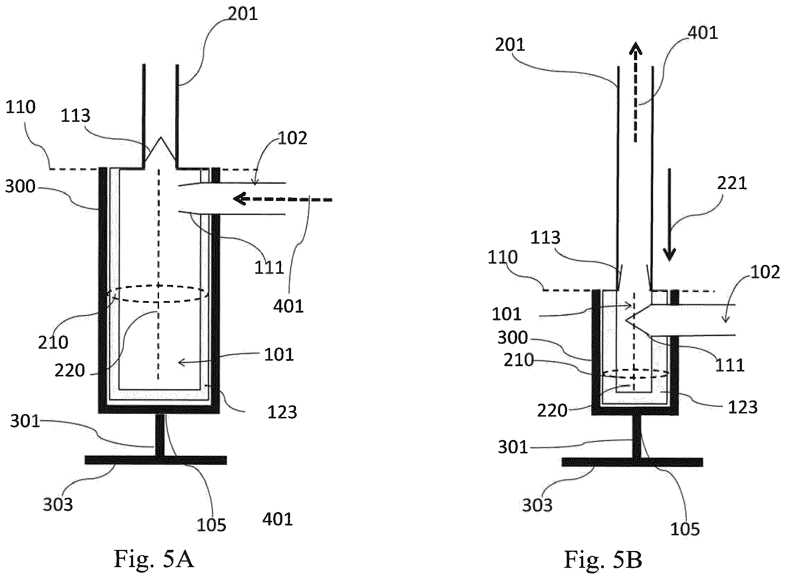

**Fig. 5A** zeigt schematisch einen linken Ventrikel 101 in der Diastole. Der Herzmuskel 123 ist entspannt, die Längsachse 220 ist am längsten, der Umfang 210 des Ventrikels 101 und der orthogonale Diameter sind am größten im Herzzyklus. Der Ventrikel 101 ist gefüllt und die Mitralklappe 111 ist geöffnet, um den Bluteinstrom 401 in den Ventrikel zu ermöglichen. Die Aortenklappe 113 ist geschlossen und verhindert so den Rückfluss von Blut aus dem Körperkreislauf in den linken Ventrikel 101 und erhält den diastolischen Blutdruck im Körper aufrecht. Die Aortenwurzel 201 ist am Ende der Diastole maximal zusammengezogen.

**Fig. 5B** zeigt schematisch die Kontraktion des Herzens mit einer normal elastischen Aortenwurzel 201. Der Herzmuskel 123 ist kontrahiert, das Herz hat den geringsten Umfang 210 bzw. orthogonalen Diameter. Die Herzspitze 105 verbleibt stationär, da der Herzbeutel 300 über das sterno-perikardiale Ligament 301 mit dem Sternum 303 verbunden ist. Aufgrund der Kontraktion des Herzmuskels 123 hat sich die Herzbasis 110 mit der Bewegungsrichtung 221 zur Herzspitze 105 verlagert und die Längsachse 220 ist am kürzesten im Herzzyklus. Die Mitralklappe 111 ist geschlossen, um den Rückfluss von Blut in das Atrium 102 zu verhindern. Die Aortenklappe 113 ist geöffnet, um den Auswurf von Blut 401 in den Körperkreislauf zu ermöglichen. Die Aortenwurzel 201 ist maximal gestreckt.

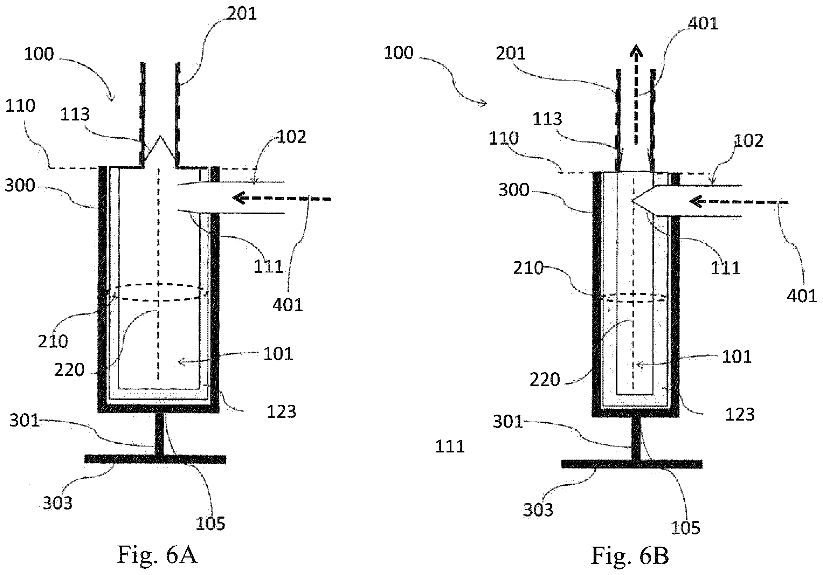

**Fig. 6A** zeigt schematisch einen linken Ventrikel 101 in der Diastole jedoch bei steifer, nicht elastischer Aortenwurzel 201. Die Situation ist im Wesentlichen identisch mit Fig. 5A. Der Herzmuskel 123 ist entspannt, die Längsachse 220 ist am längsten, der Umfang 210 des Ventrikels 101 und der orthogonale Diameter sind am größten im Herzzyklus. Der Ventrikel 101 ist gefüllt und die Mitralklappe 111 ist geöffnet, um den Bluteinstrom 401 in den Ventrikel zu ermöglichen. Die Aortenklappe 113 ist geschlossen und verhindert so den Rückfluss von Blut aus dem Körperkreislauf in den linken Ventrikel 101 und erhält den diastolischen Blutdruck im Körper aufrecht. Die Aortenwurzel 201 ist am Ende der Diastole maximal zusammengezogen.

**Fig. 6B** zeigt schematisch die Kontraktion des linken Ventrikels 101 bei steifer, nicht elastischer Aortenwurzel 201. Der Herzmuskel 123 ist kontrahiert. Die Herzspitze 105 verbleibt stationär, da der Herzbeutel 300 über das sterno-perikardiale Ligament 301 mit dem Sternum 303 verbunden ist. Die Lage der Herzbasis 110 hat sich nicht verändert, da die steife Aortenwurzel 201 nicht gedehnt werden kann und so die Herzbasis nicht zur Herzspitze hingezogen werden kann. Die Längsachse 220 ist ebenso lang wie bei entspanntem Herzen in der Diastole in **Fig. 4A****.** Aufgrund der Muskelkontraktion ist hier jedoch der Umfang 210 und der orthogonale Diameter am geringsten, was den gesamten Herzzyklus betrifft. Die Mitralklappe 111 ist geschlossen, um den Rückfluss von Blut in das linke Atrium 102 zu verhindern. Die Aortenklappe 113 ist geöffnet, um den Auswurf von Blut in den Körperkreislauf zu ermöglichen.

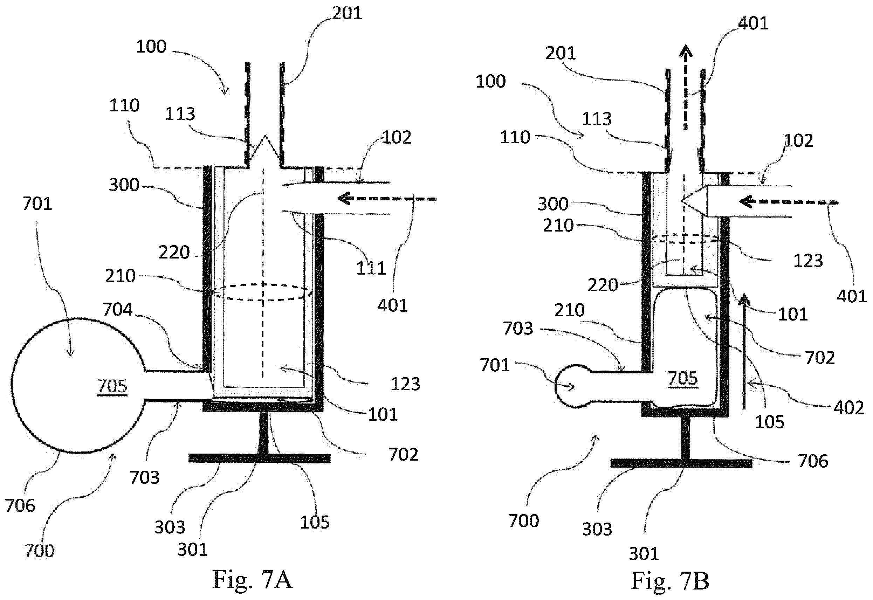

**Fig. 7A und 7B** zeigen die Funktion einer Ausführungsform des erfindungsgemäßen Kammersystems 700 mit Bezug auf einen schematisch angedeuteten Herzbeutel 300 eines schematisch angedeuteten Herzens 100.

**Fig. 7A** zeigt eine schematische Darstellung des Herzens 100 in der Diastole. Das erfindungsgemäße Kammersystem 700 weist eine zweite Kammer 701 außerhalb des Herzbeutels 300, eine erste Kammer 702 innerhalb des Herzbeutels 300 und den Verbindungskanal 703 zwischen den Kammern 701 und 702 auf oder besteht hieraus, wobei der Verbindungskanal durch eine Öffnung im Herzbeutel 704 hindurchtritt.

Die erste Kammer 702 ist geschrumpft und erlaubt so eine maximale Ausdehnung des Herzens 100 und eine maximale Füllung der Herzkammer 101 in der Diastole. Das im Kammersystem 700 vorgesehene Fluid befindet sich fast vollständig in der zweiten Kammer 701.

**Fig. 7B** zeigt eine Ausführungsform des erfindungsgemäßen Kammersystems 700 im Herzbeutel 300 mit Herz 100. Dabei zeigt **Fig. 7B** eine schematische Darstellung des Herzens 100 in der Systole und das erfindungsgemäße Kammersystem 700 mit der zweiten Kammer 701 außerhalb des Herzbeutels 300, der ersten Kammer 702 im Herzbeutel 300 und dem Verbindungskanal 703 zwischen den Kammern 701 und 702. Der Verbindungskanal 703 tritt durch ein Loch oder eine Öffnung im Herzbeutel 704 hindurch. Die erste Kammer 702 ist ausgedehnt und erlaubt so eine Verlagerung der Herzspitze 105 in Bewegungsrichtung 402 hin zur Herzbasis 110. Sie erlaubt somit eine maximale Verkürzung der langen Achse 220 der Herzkammer 101 und somit eine maximale Entleerung der Herzkammer 101. Das Fluid des Kammersystems 700 befindet sich fast vollständig in der ersten Kammer 702, und die zweite Kammer 701 ist geschrumpft oder kleiner geworden.

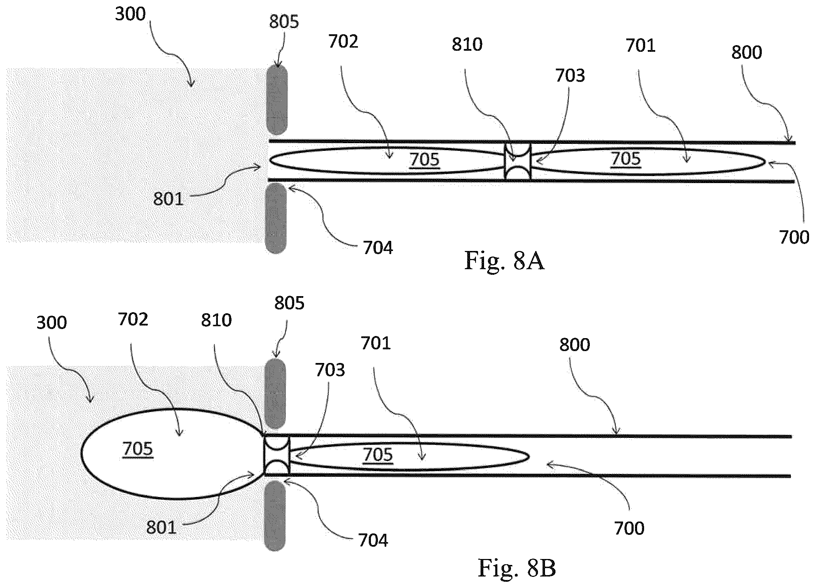

**Fig. 8A bis 8D** zeigen das Einbringen des erfindungsgemäßen Kammersystems 700.

**Fig. 8A** zeigt das erfindungsgemäße Kammersystem 700, vollständig aufgenommen in einem Einführungskatheter 800. Die Spitze 801 des Einführungskatheters 800 hat die Herzbeutelwand 805 mittels der Öffnung 704 überwunden und liegt innerhalb des Herzbeutels vor.

**Fig 8B** zeigt das Kammersystem 700 in Richtung Spitze 801 des Einführungskatheters 800 vorgeschoben, so dass die erste Kammer 702 den Einführungskatheter 800 durch dessen endseitiges Lumen verlassen und sich im Herzbeutel 300 ausgedehnt hat.

Der Verbindungskanal 703, welcher ebenfalls elastisch oder entfaltbar ausgestaltet sein kann, steckt in der in Fig. 8b gezeigten Phase der Implantation noch im Lumen des Einführungskatheters 800 und wird durch dessen Innenwände an einem radialen Entfalten gehindert.

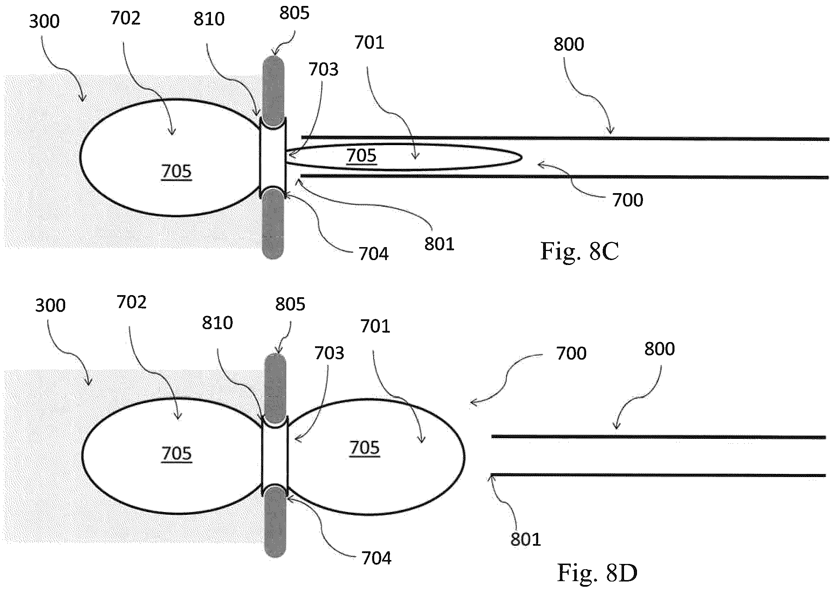

**Fig. 8C** zeigt die Spitze 801 des Einführungskatheters 800 weiter zurückgezogen. Der Verbindungskanal 703 und der Stent 810 liegen außerhalb des Lumens des Einführungskatheters 800 und haben sich entfaltet, etwa durch ein Formgedächtnis des Stents 810, und haben die Öffnung 704 im Herzbeutel 300 radial vergrößert. Der Stent 810 ist in der Herzbeutelwand 805 verankert oder hält sich an dieser, etwa durch radiale Kräfte, fest.

**Fig. 8D** zeigt den Einführungskatheter 800 weiter zurückgezogen, so dass sich auch die zweite Kammer 701 ausgedehnt hat, und zwar außerhalb des Herzbeutels 300.

Das Kammersystem 700 liegt - bezogen auf diese Ausführungsform - somit vollständig außerhalb des Einführungskatheters 800 vor. Letzter wird nicht mehr benötigt und kann verworfen werden.

Zum Ausbringen des Kammersystems 700 aus dem Einführungskatheter 800 heraus kann ein Werkzeug zum Einsatz kommen, das hier nicht gezeigt ist, z. B. ein sog. Pusher.

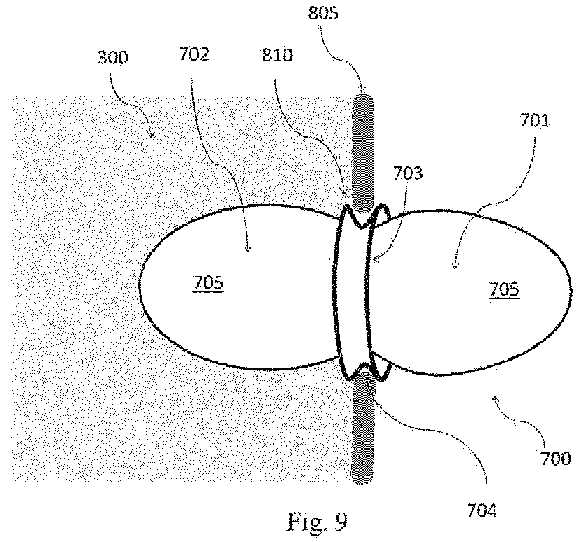

**Fig. 9** zeigt eine perspektivische, dreidimensionale Darstellung des Stents 810, der sich in der Öffnung der Herzbeutelwand 805 entfaltet hat und verankert ist. Beide Kammern 701 und 702 sind ausgedehnt.

Die Kammern 701 und 702 haben ein Volumen von 0 ml - 500 ml, besonders vorzugsweise ein Volumen von 0,01 ml - 300 ml, weiter vorzugsweise ein Volumen von 0,10 ml - 200 ml, ganz besonders vorzugsweise ein Volumen von 1,00 ml - 100 ml.

Der Anteil des Fluids, der zwischen den Kammern 701 und 702 bewegt wird hat einen Anteil am Gesamtvolumen des Fluids von 0,1 % - 20 %, besonders vorzugsweise einen Anteil am Gesamtvolumen des Fluids von 1 % - 50 %, besonders vorzugsweise einen Anteil am Gesamtvolumen des Fluids von 10 % - 90 %.

Der Verbindungskanal 703, der die Kammern 701, 702 miteinander verbindet, hat vorzugsweise einen Durchmesser von 0,5 cm - 5 cm. Besonders vorzugsweise einen Durchmesser von 15 mm - 20 mm.

Der Verbindungskanal 703, der die Kammern 701, 702 miteinander verbindet, hat vorzugsweise eine Länge von 1 mm - 500 mm. Besonders vorzugsweise eine Länge von 1 mm - 100 mm. Besonders vorzugsweise eine Länge von 2 mm - 10 mm.

Das Fluid in den Kammern 701, 702 kann jedes Fluid sein, das über die notwendigen Flusseigenschaften verfügt. Vorzugsweise hat der Verbindungskanal 703, der die erste Kammer 702 im Herzbeutel mit der zweiten Kammer 701 außerhalb des Herzbeutels 300 verbindet, einen ausreichend großen Durchmesser und das Fluid eine ausreichend niedrige Viskosität, so dass bei der Verschiebung des Fluids zwischen den beiden Kammern nur geringste Energieverluste auftreten.

Die Größenverhältnisse der in den vorangegangenen Figuren gezeigten Kammern 701, 702 dient rein der Anschaulichkeit. Die dargestellten Verhältnisse zwischen den Kammervolumen sind ohne Bedeutung.

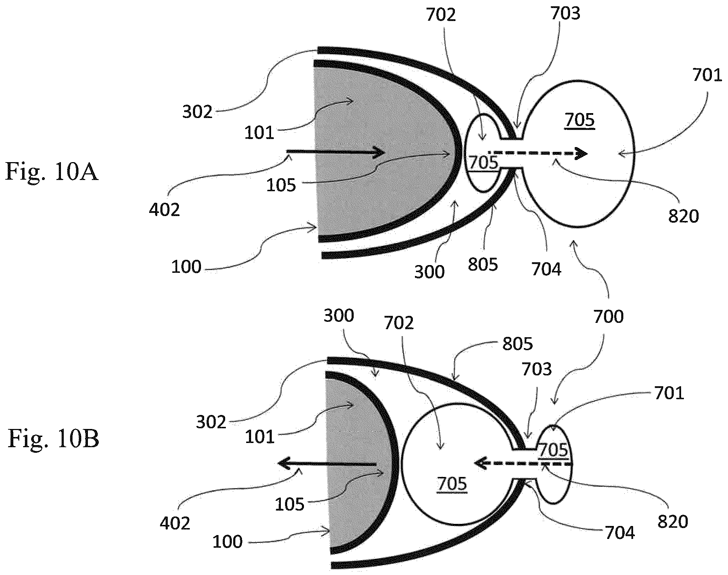

**Fig. 10A und 10B** zeigen schematisch die Funktion einer Ausführungsform des erfindungsgemäßen Kammersystems 700 mit Herzbeutel 300 und mit Herz 100.

**Fig. 10A** zeigt schematisch eine Darstellung des Herzens 100 am Ende der Diastole und des erfindungsgemäßen Kammersystems 700 mit einer Kammer 701 außerhalb des Herzbeutels 300, einer Kammer 702 im Herzbeutel 300 und dem Verbindungskanal 703 zwischen der Kammer 701 und 702. Wobei der Verbindungskanal durch eine Öffnung 704 in der Herzbeutelwand 805 hindurchtritt. Durch die Füllung der Herzkammer 101 in der Diastole wirkt die Herzspitze 105, dargestellt durch den Pfeil 402, auf die zweite Kammer 702 ein, die sich dadurch verkleinert und so eine maximale Verlängerung der Längsachse der Herzkammer 101 und eine maximale Füllung der Herzkammer 101 erlaubt. Das Fluid 705 bewegt sich dadurch, dargestellt durch Pfeil 820, von der ersten Kammer 702 in die zweite Kammer 701.

**Fig. 10B** zeigt schematisch eine Darstellung des Herzens 100 am Ende der Systole und des erfindungsgemäßen Kammersystems 700, welches mit der zweiten Kammer 701 außerhalb des Herzbeutels 300 und mit der ersten Kammer 702 im Herzbeutel 300 vorliegt, wobei der Verbindungskanal 703 zwischen den Kammern 701 und 702 liegt, wobei der Verbindungskanal durch die Öffnung 704 in der Herzbeutelwand 805 hindurchtritt.

Durch die Kontraktion der Herzkammer 101 in der Systole bewegt sich die Herzspitze 105 dargestellt durch den Pfeil 402 von der Kammer 702 weg, die sich dadurch vergrößert und so eine maximale Verkürzung der Längsachse der Herzkammer 101 und maximale Entleerung der Herzkammer 101 erlaubt. Das Fluid 705 bewegt sich dadurch, dargestellt durch Pfeil 820 von der zweiten Kammer 701 zur ersten Kammer 702.

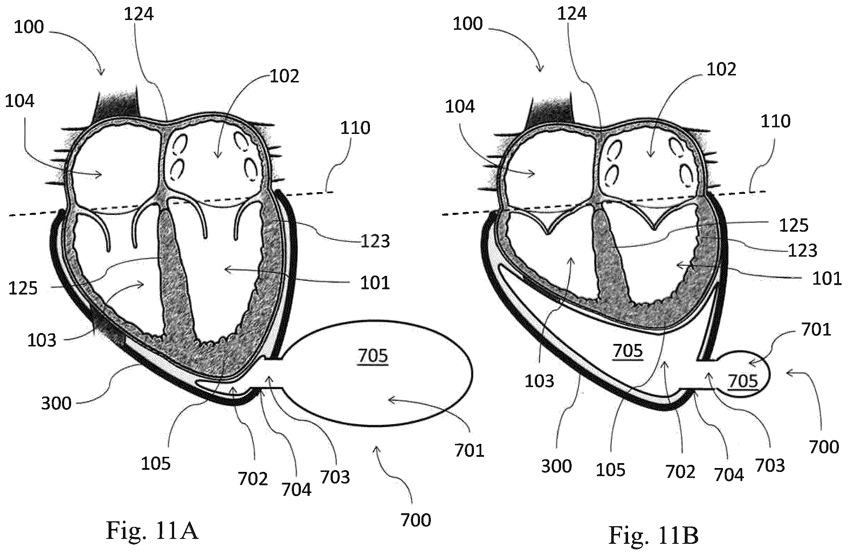

**Fig. 11A und 11B** zeigen die Funktion einer Ausführungsform des erfindungsgemäßen Kammersystems 700, implantiert in den Herzbeutel 300 bzw. das Herz 100. Die Herzbasis 110 bleibt während des gesamten Herzzyklus stationär. Durch Einlage des erfindungsmäßigen Kammersystems 700 mit ihrer ersten Kammer 702 innerhalb des Herzbeutels 300 ist die Herzspitze mobil und kann sich in der Systole zur Herzbasis 110 hinbewegen und in der Diastole von der Herzbasis 110 wegbewegen.

**Fig. 11A** zeigt eine Darstellung des Herzens 100 am Ende der Diastole. Die lange Achse des Herzens ist hier am längsten, die Herzkammern 101 und 103 füllen den Herzbeutel 300 fast vollständig aus. Das erfindungsgemäße Kammersystem 700 befindet sich mit ihrer zweiten Kammer 701 außerhalb des Herzbeutels 300, ihrer ersten Kammer 702 im Herzbeutel 300. Der Verbindungskanal 703 ist im Lumen des Herzbeutels 704 hindurchtritt. Durch die Füllung der Herzkammern 101 und 103 in der Diastole ist die erste Kammer 702 geschrumpft und erlaubt so eine maximale Füllung der Herzkammer 101 und 103. Das Fluid 705 befindet sich vorwiegend in der zweiten Kammer 701. Die erste Kammer 702 hat sich den anatomischen Gegebenheiten innerhalb des Herzbeutels 300 und der anatomischen Form der Herzspitze 105 angepasst. Die zweite Kammer 701, die sich in dieser Ausführung in der linken Brusthöhle befindet hat sich den anatomischen Gegebenheiten des nicht dargestellten Zwerchfells und der nicht dargestellten Lunge angepasst.

**Fig. 11B** zeigt eine Darstellung des Herzens 100 gegen Ende der Systole. Der Herzmuskel 123 mit dem interventrikulären Septum 125 ist kontrahiert, die lange Achse des Herzens ist hier am kürzesten. Die Herzspitze 105 hat sich in Richtung Herzbasis 110 verlagert. Die Herzkammern 101 und 103 haben sich maximal geleert. Das erfindungsgemäße Kammersystem 700 befindet sich unverändert mit der zweiten Kammer 701 außerhalb des Herzbeutels 300 und mit der ersten Kammer 702 im Herzbeutel 300. Durch Kontraktion der Herzkammern 101 und 103 in der Systole ist die erste Kammer 702 ausgedehnt und erlaubt so die Verlagerung der Herzspitze 105 hin zur Herzbasis 110 und somit eine maximale Entleerung der Herzkammern 101 und 103. Das Fluid 705 befindet sich vorwiegend in der ersten Kammer 702. Die erste Kammer 702 hat sich den anatomischen Gegebenheiten im Herzbeutel 300 und der anatomischen Form der Herzspitze 105 angepasst. Die zweite Kammer 701, die sich in dieser Ausführung in der linken Brusthöhle befindet, hat sich den anatomischen Gegebenheiten des nicht dargestellten Zwerchfells und der nicht dargestellten Lunge angepasst.

Das erfindungsgemäße Kammersystem 700 kann in einigen Ausführungsformen vollständig chirurgisch über eine offene Herzoperation oder eine seitliche Öffnung des Brustkorbes oder über eine Öffnung des Bauchraumes implantiert werden. Alternativ kann das erfindungsgemäße Kammersystem 700 in einigen Ausführungsformen über einen Kombinationseingriff mittels Katheter und chirurgischem Eingriff implantiert werden. Alternativ kann das erfindungsgemäße Kammersystem 700 in einigen Ausführungsformen auch vollständig mittels Katheter und minimal invasiven Eingriff im Bereich des Herzens, im Bereich beider Brusthöhlen oder im Bereich der Bauchhöhle eingebracht werden.

Das Einbringen des erfindungsgemäßen Kammersystems 700 mittels Katheter erfolgt vorzugsweise mit dem Einführungskatheter 800. Die Spitze 801 des Einführungskatheters 800 wird je nach Notwendigkeit in die Herzhöhle, den Brustkorb oder den Bauchraum eingeführt. Mittels eines üblicherweise verwendeten Katheters und Trokars wird eine Öffnung 704 in dem Herzbeutel erzeugt und mittels Dilatoren und Dilatationsballon vergrößert. Die Spitze 801 des Einführungskatheters 800 wird in den Herzbeutel 300 vorgeschoben, so dass die erste Kammer 702 den Einführungskatheter 800 verlassen und sich im Herzbeutel 300 ausdehnen kann. Daraufhin wird die Spitze 801 des Einführungskatheters 800 zurückgezogen, so dass sich der Verbindungskanal 703 mit dem Stent 810 in dem die Öffnung 704 im Herzbeutel entfaltet, dabei vergrößert und stabilisiert. Der Stent 810 ist sodann in der Herzbeutelwand 805 verankert. Der Einführungskatheter 800 wird weiter zurückgezogen, so dass sich auch die zweite Kammer 701 ausdehnen kann.

### Bezugszeichen

- 100: Herz
- 101: linker Ventrikel, linke Herzkammer
- 102: linkes Atrium, linker Vorhof
- 103: rechter Ventrikel, rechte Herzkammer
- 104: rechtes Atrium, rechter Vorhof
- 105: Herzspitze
- 110: Herzbasis
- 111: Mitralklappe
- 112: Trikuspidalklappe
- 113: Aortenklappe
- 114: Pulmonalklappe
- 120: Herzskelett
- 121: linkes fibröses Trigon
- 122: rechtes fibröses Trigon
- 123: Herzmuskel
- 124: atriales Septum, Vorhofseptum
- 125: interventrikuläres Septum, Herzkammerscheidewand
- 131: Mitralklappenring
- 132: Trikuspidalklappenring
- 201: Aortenwurzel
- 210: Umfang der Herzkammern in der Diastole, orthogonaler Diameter
- 220: Längsachse der Herzkammern
- 221: Bewegungsrichtung der Herzbasis
- 300: Perikard, Herzbeutel
- 301: sterno-perikardiales Ligament
- 302: Diaphragma, Zwerchfell
- 303: Sternum
- 304: Processus xiphoideus
- 305: Rippe
- 306: Mediastinum, Mittelfellraum
- 401: Blutstrom
- 402: Bewegungsrichtung der Herzspitze
- 700: Medizinisches Kammersystem
- 701: zweite Kammer außerhalb des Herzbeutels
- 702: erste Kammer im Herzbeutel
- 703: Verbindungskanal zwischen den Kammern
- 704: Öffnung; Loch im Herzbeutel
- 705: Fluid
- 706: Kammerwand
- 800: Einführungskatheter
- 801: Spitze des Einführungskatheters
- 805: Herzbeutelwand
- 810: Stützvorrichtung; Stent
- 820: Flussrichtung des Fluids

## Patentansprüche

1. Medizinisches Kammersystem (700) zur Implantation in den Brustkorb eines Patienten zur Unterstützung der Herzaktion, vorzugsweise durch Verlagerung der Herzspitze (105), umfassend wenigstens eine erste Kammer (702) zum Anordnen innerhalb des Herzbeutels (300) und eine zweite Kammer (701) zum Anordnen außerhalb des Herzbeutels (300), wobei die Kammern (701, 702) wenigstens einen Verbindungsabschnitt oder einen
Verbindungskanal (703) aufweisen, der die beiden Kammern (701, 702) miteinander verbindet, wobei die Kammern (701, 702) und der Verbindungskanal (703) ferner ausgestaltet sind, um mit Fluid (705) befüllt zu werden und um vorzugsweise im implantierten Zustand derart angeordnet zu sein oder zu werden, dass die Herzaktion auf die erste Kammer (702) einwirkt und die zweite Kammer (701) als Volumenspeicher und/oder Energiespeicher für das Fluid (705) wirkt;
**dadurch gekennzeichnet, dass**
das Kammersystem eine Stützvorrichtung (810) umfasst,
wobei die Stützvorrichtung (810) den Verbindungskanal (703) zumindest abschnittsweise ausformt oder ihm Struktur gibt, und
wobei die Stützvorrichtung ein Hohlkörper ist, und wobei die erste Kammer (702) und die zweite Kammer (701) gemeinsam mit dem Verbindungskanal (703) genau einen geschlossenen Beutel ausbilden.

2. Medizinisches Kammersystem (700) nach Anspruch 1, wobei eine Kammerwand (706) des Kammersystems (700) zumindest abschnittsweise flexibel verformbar ist.

3. Medizinisches Kammersystem (700) nach einem der vorangegangenen Ansprüche, wobei die Stützvorrichtung (810) eine flexibel verformbare Stützvorrichtung (810), insbesondere ein Stent (810), oder ein Ring oder ein Zylinder ist.

4. Medizinisches Kammersystem (700) nach einem der vorangegangenen Ansprüche, wobei das Füllvolumen der Kammern (701, 702) zusammen zwischen 0,1 ml und 100 ml, insbesondere zwischen 1 ml und 50 ml beträgt.

5. Medizinisches Kammersystem (700) nach einem der vorangegangenen Ansprüche, wobei das Kammersystem (700) Sensoren, insbesondere Drucksensoren und/oder Lagesensoren umfasst.

6. Kit, umfassend ein medizinisches Kammersystem (700) nach einem der Ansprüche 1 bis 5 und ein Einführsystem für das medizinische Kammersystem (700), wobei das Einführsystem einen Einführungskatheter (800), einen Führungskatheter, einen Führungsdraht und/oder mindestens einen Abgabekatheter umfasst.

## Claims

1. A medical chamber system (700) for implantation into the thorax of a patient to support cardiac action, preferably by displacing the cardiac apex (105), comprising at least a first chamber (702) for arrangement inside the pericardium (300) and a second chamber (701) for arrangement outside the pericardium (300), wherein the chambers (701, 702) have at least one connecting section, or a connecting channel (703), connecting the two chambers (701, 702) to each other, the chambers (701, 702) and the connecting channel (703) being further configured to be filled with fluid (705) and preferably, in the implanted state, to be arranged such that the cardiac action acts on the first chamber (702) and the second chamber (701) acts as a volume reservoir and/or energy storage for the fluid (705);
**characterized in that**
the chamber system comprises a support device (810),
wherein the support device (810) forms the connecting channel (703) at least in sections or provides structure to it, and wherein the support device is a hollow body,
and wherein the first chamber (702) and the second chamber (701) together with the connecting channel (703) form exactly one closed bag.

2. The medical chamber system (700) according to claim 1, wherein a chamber wall (706) of the chamber system (700) is flexibly deformable at least in sections.

3. The medical chamber system (700) according to any one of the preceding claims, wherein the support device (810) is a flexibly deformable support device (810), in particular a stent (810), or a ring or a cylinder.

4. The medical chamber system (700) according to any one of the preceding claims, wherein the combined filling volume of the chambers (701, 702) is from 0.1 ml to 100 ml, in particular from 1 ml to 50 ml.

5. The medical chamber system (700) according to any one of the preceding claims, wherein the chamber system (700) comprises sensors, in particular pressure sensors and/or position sensors.

6. A kit comprising a medical chamber system (700) according to any one of claims 1 to 5 and an insertion system for the medical chamber system (700), wherein the insertion system comprises an insertion catheter (800), a guide catheter, a guidewire and/or at least one delivery catheter.

## Revendications

1. Un système de chambres médical (700) destiné à être implanté dans le thorax d'un patient pour assister l'action cardiaque, de préférence par déplacement de l'apex cardiaque (105), comprenant au moins une première chambre (702) destinée à être disposée à l'intérieur du péricarde (300) et une deuxième chambre (701) destinée à être disposée à l'extérieur du péricarde (300), les chambres (701, 702) présentant au moins une section de liaison, ou un canal de liaison (703), qui relie les deux chambres (701, 702) l'une à l'autre,
les chambres (701, 702) et le canal de liaison (703) étant en outre conçus pour être remplis de fluide (705) et pour être disposés, de préférence à l'état implanté, de telle sorte que l'action cardiaque agisse sur la première chambre (702) et que la deuxième chambre (701) agisse comme un réservoir de volume et/ou un accumulateur d'énergie pour le fluide (705);
**caractérisé en ce que**
le système de chambres comprend un dispositif de support (810), lequel dispositif de support (810) forme au moins partiellement le canal de liaison (703) ou lui confère une structure, le dispositif de support étant un corps creux, et la première chambre (702) et la deuxième chambre (701) formant, conjointement avec le canal de liaison (703), exactement une poche fermée.

2. Le système de chambres médical (700) selon la revendication 1, dans lequel une paroi de chambre (706) du système de chambres (700) est déformable de manière flexible au moins partiellement.

3. Le système de chambres médical (700) selon l'une quelconque des revendications précédentes, dans lequel le dispositif de support (810) est un dispositif de support (810) déformable de manière flexible, notamment un stent (810), ou un anneau ou un cylindre.

4. Le système de chambres médical (700) selon l'une quelconque des revendications précédentes, dans lequel le volume de remplissage total des chambres (701, 702) est compris entre 0,1 ml et 100 ml, notamment entre 1 ml et 50 ml.

5. Le système de chambres médical (700) selon l'une quelconque des revendications précédentes, dans lequel le système de chambres (700) comprend des capteurs, notamment des capteurs de pression et/ou des capteurs de position.

6. Un kit comprenant un système de chambres médical (700) selon l'une quelconque des revendications 1 à 5 et un système d'introduction pour le système de chambres médical (700), dans lequel le système d'introduction comprend un cathéter d'introduction (800), un cathéter de guidage, un fil-guide et/ou au moins un cathéter d'administration.
